# FASCICULE DE BREVET EUROPEEN

(11) **EP 1 397 104 B1**
(45) Date de publication et mention de la délivrance du brevet: **28.04.2010**
(21) Numéro de dépôt: 02745506.2
(22) Date de dépôt: 10.06.2002
(51) Int. Cl.: A61J 1/00

(54) **Procédé et dispositif de suivi du veillissement des poches de sang**
Verfahren und Vorrichtung zur Überwachung der Alterung von Blutbeuteln
Method for determining and monitoring ageing of blood bags

(30) Priorité: 12.06.2001 FR 0107618
(43) Date de publication de la demande: 17.03.2004
(73) Titulaire: Biolog, 92517 Boulogne Billancourt (FR)
(72) Inventeur: DE GAULLE, Antoine, F-92310 Sevres (FR); MONGRENIER, Jean-Claude, F-78100 Saint Germain en Laye (FR)
(74) Mandataire: Schmitt, John
(86) Numéro de dépôt international: PCT/FR2002/001968
(87) Numéro de publication internationale: WO 2002/100462

(56) Documents cités:
- WO-A-00/45331
- FR-A- 2 796 182
- US-A- 5 969 606

## Description

L'invention concerne un procédé de détermination et de suivi du vieillissement des poches de sang dans les établissements de transfusion sanguine et les établissements de soins.

Actuellement les poches de sang sont prélevées dans les établissements de transfusion sanguine chaque poche est datée au moment du prélèvement ce qui marque l'origine de la vie de la poche et des produits dérivés pour une durée prédéterminée; les poches de sang prélevées subissent une filtration, une centrifugation et une séparation qui aboutit notamment à un produit dérivé qui est une poche de globules rouges destinée à être transfusée dont la durée de vie prédéterminée est de quarante cinq jours à partir du prélèvement de sang; les poches de globules rouges sont stockées dans les établissements de transfusion sanguine et délivrées au fur et à mesure des besoins des établissements de soins. Il arrive que des poches de globules rouges qui avaient été prévues pour pallier un incident en cours d'opération n'aient pas été utilisées ; comme il est impossible d'avoir actuellement une garantie certaine de la qualité du contenu de la poche de globules rouges, en vue d'une nouvelle utilisation, celle-ci est détruite. La destruction des poches de globules rouges non utilisées représentant actuellement une perte de douze pour cent, des poches de sang prélevées, qu'il est indispensable de réduire.

Les poches de globules rouges étant actuellement transportées entre l'établissement de transfusion sanguine et l'établissement de soins sans précautions particulières, une étude est en cours avec une société de transport pour garantir la continuité de la chaîne de froid entre ces deux établissements ; la poche de sang est ainsi placée dans un conteneur réfrigéré à l'intérieur duquel est placé un dispositif indiquant la température dans le conteneur tout au cours du transport; les poches de globules rouges, étant placées à l'arrivée dans un réfrigérateur approprié de l'établissement de soins, peuvent être retournées à l'établissement de transfusion sanguine si entre temps elles n'ont pas été extraites pour être mises à la disposition d'un chirurgien.

Lors de la prise de sang, le sang est transféré dans une poche de sang mère reliée à un filtre lui-même relié à une poche primaire de sang ; la poche de sang primaire est solidaire d'un groupe de trois poches secondaires auxquelles elle est reliée par des tubulures souples branchées en parallèle; le sang de la poche de sang mère est filtré et introduit dans la poche de sang primaire; la poche de sang primaire est centrifugée pour séparer les globules rouges, des plaquettes et du sérum qui sont ensuite respectivement transférés dans chacune des trois poches et c'est la poche contenant les globules rouges qui est utilisée pour réaliser les transfusions sanguines. Pour mémoire, les plaquettes ne sont isolées que lorsqu'il y a un besoin spécifique à approvisionner ; sinon elles restent avec les globules rouges et il n'y a que deux poches secondaires utilisées.

Un dispositif de tracabilité des poches de sang, selon la demande de brevet FR 2825637 est en cours de développement il associe une puce électronique à la poche de sang; chaque puce électronique comporte une antenne annulaire qui communique avec l'antenne annulaire d'un dispositif de communication électronique, relié à un dispositif informatique, susceptible de fournir à la puce électronique d'une part de l'énergie et d'autre part des informations qu'elle mémorise et qu'elle est susceptible de restituer audit dispositif informatique par l'intermédiaire du dispositif de communication électronique; une puce électronique mère est fixée sur la poche de sang mère qui recueille toutes les informations sur le donneur et les résultats des analyses permettant la qualification de la poche de sang mère; la puce électronique mère est fixée sur un support de puce souple de forme rectangulaire de quelques centimètres de côté sur lequel est imprimé un circuit métallisé en boucles formant l'antenne annulaire de communication; dans une version préférée de l'invention, le support de puce souple de la puce électronique primaire est placé sur une des grandes faces de la poche de sang mère et sous une étiquette rectangulaire recouvrant la plus grande partie d'une face principale; de préférence les supports de puce mère sont toujours placés au même endroit par rapport à l'étiquette de manière à faciliter le positionnement de l'antenne du dispositif de communication électronique. Une demande de brevet FR-2796182 décrit un peson-agitateur muni d'un dispositif de communication électronique permettant d'enregistrer dans la puce électronique mère de la poche de sang mère les caractéristiques du donneur de sang et les conditions de prélèvement ; le sang de la poche de sang mère est transféré par filtration dans la poche de sang primaire; la poche de sang primaire comporte une puce électronique primaire dans laquelle sont transférées les informations contenues dans la puce électronique mère ainsi que les informations concernant les conditions de filtration; la poche de sang primaire est alors centrifugée; les constituants sont séparés et sont introduits dans les poches secondaires de sang; les poches secondaires de sang sont équipées de puces électroniques secondaires, identiques aux puces électroniques mères et primaires, qui sont fixées sur un support de puce souple placé sous une étiquette recouvrant une face de la poche secondaire de sang et de préférence à une place telle que, lorsque les poches mère, primaire et secondaire de sang sont superposées, les supports de puce électronique mère et primaire ne soient pas superposés entre eux et aux supports de puces souples secondaires; les puces électroniques secondaires, des poches secondaires, c'est-à-dire la poche de globules rouges, la poche de sérum et éventuellement la poche de plaquettes, sont renseignées par le transfert des informations de la puce électronique primaire, de la poche de sang primaire, complétées par les informations concernant les paramètres utilisés pour la séparation des composants sanguins. En ce qui concerne la poche de globules rouges ayant servi à une transfusion sanguine, la puce électronique secondaire qui lui est associée comporte en fin d'utilisation les informations concernant les conditions de son utilisation et notamment l'identité du malade transfusé.

Le procédé, objet de l'invention, consiste à formaliser tout au cours de son parcours l'état d'une poche de sang mère, primaire ou secondaire, qu'on appelle ci-après "poche de sang", équipée d'une puce électronique, vis à vis du phénomène de biodégradation qu'on qualifie ci-après de "vieillissement", de manière que l'on sache à tout moment de manière la plus précise possible, si la poche de sang est qualifiée pour être transfusée.

La figure 1 représente un schéma de principe des diverses étapes susceptibles d'être parcourues par une poche de sang, sur lequel porte le procédé objet de l'invention.

La figure 2 représente une vue partielle éclatée d'un dispositif de stockage des poches de sang équipé d'un dispositif automatique de contrôle du vieillissement des poches de sang.

Les figure 3A et 3B représentent un autre mode de réalisation du dispositif de stockage selon la figure 2.

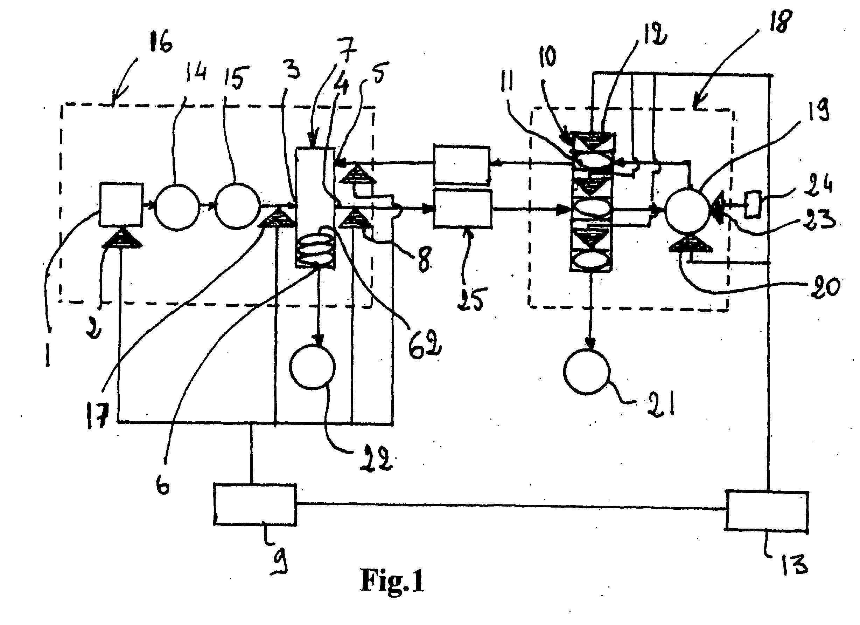

Le procédé consiste à déterminer un indice de vieillissement de la poche de sang, qui caractérise la qualification ou la déqualification pour que la poche de sang soit ou non transfusable à un patient ; la poche de sang est alors dite ci-après "qualifiée" ou "déqualifiée"; l'indice de vieillissement est calculé en mettant la puce électronique de la poche de sang en relation avec un dispositif de communication électronique lui-même relié à un ordinateur contenant des logiciels de calcul appropriés; cet indice évolue progressivement en fonction notamment du temps et des conditions environnementales vers une valeur maximale qui, lorsqu'elle est atteinte, entraîne la déqualification et la destruction de la poche de sang; la variation de cet indice de vieillissement est calculée entre deux instants successifs constituant une période, en utilisant une modélisation, appelée ci-après "modèle de vieillissement préétabli", caractérisant la période considérée et qui est établie expérimentalement à partir de formules empiriques prenant en compte les résultats d'analyses bactériologiques, les caractéristiques de l'environnement de la poche de sang pendant la période considérée et des paramètres tels que le temps et ou la température et la valeur de l'indice du vieillissement au début de la période; cet indice de vieillissement est initié au moment de la prise de sang qui indique une date qui est inscrite dans la puce électronique de la poche de sang primaire; cela suppose que le peson-agitateur 1 (fig.1) supportant la poche de sang soit équipé d'un premier dispositif de communication électronique 2 permettant d'inscrire dans la puce électronique équipant la poche de sang primaire la date et l'heure du prélèvement, en plus des informations caractéristiques concernant le donneur et les conditions du prélèvement; l'indice de vieillissement est calculé chaque fois que la poche de sang change d'environnement comme lors de l'entrée 3 et 5 et de la sortie 4 et 6 d'une zone de stockage, dont les paramètres atmosphériques sont contrôlés, dit ci-après "enceinte à atmosphère contrôlée" 7 et la nouvelle valeur de l'indice de vieillissement est inscrite dans la puce électronique de la poche de sang et la poche de sang est dite "requalifiée" si l'indice de vieillissement est inférieur à sa valeur maximale, sinon elle est déqualifiée; une telle opération se réalise manuellement au moment où l'on extrait la poche de sang d'une enceinte à atmosphère contrôlée 7 en utilisant un deuxième dispositif de communication électronique 8 relié, à un premier ordinateur 9 contenant les logiciels appropriés; une telle opération peut aussi se réaliser automatiquement dans une enceinte à atmosphère contrôlée où la puce électronique de chaque poche de sang 11 est mise en relation permanente avec un dispositif de communication électronique spécialisé 12 relié à un deuxième ordinateur 13 contenant les logiciels appropriés; ce type d'enceinte à atmosphère contrôlée est appelée ci-après "enceinte à atmosphère contrôlée cellulaire" 10; dans ces conditions la poche de sang 11 peut être prélevée à tout moment en ayant inscrit dans sa puce électronique son indice de vieillissement à jour.

A titre d'exemple, le temps initial est inscrit, lors du prélèvement sanguin, au niveau du peson-agitateur 1, dans la puce électronique primaire de la poche de sang primaire qui est ensuite traitée dans une centrifugeuse 14 ; les globules rouges sont introduits lors de la phase de séparation 15 dans une des trois poches secondaires pour constituer la poche de sang; les informations contenues dans la puce électronique primaire sont transférées dans la puce électronique de la poche de sang en même temps que les informations concernant la centrifugation et la séparation ; la poche de sang est dirigée alors vers l'enceinte à atmosphère contrôlée 7 de l'établissement de transfusion sanguine 16 et une première valeur de l'indice de vieillissement est calculée en mettant sa puce électronique en communication avec un premier ordinateur 9 par l'intermédiaire d'un troisième dispositif de communication électronique 17; en cours de stockage dans l'enceinte à atmosphère contrôlée 7 la poche de sang 62 n'est en principe pas suivie au point de vue du vieillissement si ce n'est pour des contrôles statistiques de l'état des stocks et l'indice de vieillissement est alors mis à jour pour la poche de sang 62 contrôlée; lorsque la poche de sang 62 est extraite pour une opération de transport 25. vers un établissement de soins 18, l'indice de vieillissement est mis à jour par rapport à la date de la dernière mise à jour de l'indice de vieillissement à l'aide du deuxième dispositif de communication électronique 8; les mises à jour de l'indice de vieillissement se font ensuite dans l'établissement de soins 18 successivement à l'entrée dans l'enceinte à atmosphère contrôlée cellulaire 10 et ensuite à des instants réguliers et rapprochés jusqu'à ce que la poche de sang 11 soit prélevée pour être dirigée vers une salle d'opération 19 où elle sera encore établie une dernière fois avant transfusion grâce à un quatrième dispositif de communication électronique 20; si la poche de sang n'est pas utilisée, elle est retournée dans l'enceinte à atmosphère contrôlée cellulaire 11 et même vers l'établissement de transfusion sanguine 16 en suivant une procédure analogue à la procédure précédemment décrite.

Lorsque la poche de sang 11 est dans une enceinte à atmosphère contrôlée cellulaire 10 d'un établissement de soins 18, la puce électronique associée à chaque poche de sang 11 est reliée en permanence au deuxième ordinateur 13 de l'établissement de soins 18 par l'intermédiaire d'un dispositif de communication électronique spécialisé 12 ; le deuxième ordinateur 13 contrôle à intervalle de temps régulier la présence de la poche de sang 11 dans l'enceinte à atmosphère contrôlée cellulaire 10 et la température qui y règne, afin de calculer le vieillissement de la poche de sang 10 par rapport à des tables de valeurs établies expérimentalement; le calcul du vieillissement peut se faire, par exemple, par la détermination de deux paramètres qui sont la température et le temps écoulé depuis la dernière mise à jour; il est déterminé une température moyenne qui est pondérée en fonction de sa valeur intrinsèque et en fonction de l'instant considéré par rapport à l'instant initial, déterminé sur le peson-agitateur 1 au moment de la prise de sang; cette température moyenne pondérée est ensuite intégrée par rapport au temps pour donner une valeur mesurant la variation de l'indice de vieillissement; pour effectuer la mise à jour, la valeur du nouvel indice de vieillissement est inscrite dans la puce électronique ainsi que la date et l'heure de sa détermination.

Il est déterminé expérimentalement une valeur maximum de l'indice de vieillissement qui entraîne automatiquement la déqualification et la destruction 21,22, de la poche de sang lorsque la valeur maximale est atteinte.

Lorsque la poche de sang 11 est prélevée dans une enceinte à atmosphère contrôlée cellulaire 10. la puce électronique comporte la valeur du dernier indice de vieillissement calculé et la date et l'heure à laquelle il a été fait ; si cette poche de sang 11 est mise à disposition dans une salle d'opération 19, celle-ci est équipée du quatrième dispositif de communication électronique 20 avec la puce électronique de la poche de sang et peut relever la valeur de l'indice de vieillissement à la date du dernier contrôle; comme à priori la température n'est plus maîtrisée depuis la sortie de la poche de sang de l'enceinte à atmosphère contrôlée cellulaire 10, il est établi expérimentalement une courbe moyenne d'évolution de la température en fonction du temps, participant à la constitution d'un modèle de vieillissement préétabli, permettant d'actualiser l'indice de vieillissement; si le quatrième dispositif de communication électronique 20 est relié au deuxième ordinateur 13 c'est ce dernier qui calcule le nouvel indice de vieillissement qui est validé et enregistré dans la puce électronique; si un cinquième dispositif de communication électronique 23 autonome, servant au contrôle de la poche de sang au moment de la transfusion sanguine, n'est pas relié à un ordinateur, comme ce peut être le cas lorsque la poche de sang est transfusée en cours de transport d'une personne accidentée, il peut être équipé d'un moyen de calcul simplifié 24 permettant de savoir si la poche est toujours qualifiée sans toutefois recalculer l'indice de vieillissement; en effet, il peut être défini une marge de sécurité au niveau de l'indice de vieillissement, lorsque ce dernier approche de sa valeur maximum, caractérisé par une durée de vie résiduelle de la poche de sang en atmosphère non contrôlée; si la poche de sang 11 a entamé en cours de séjour dans l'enceinte à atmosphère contrôlée cellulaire 10 sa durée de vie résiduelle, elle est déqualifiée et envoyée à la destruction 21; si le dispositif de communication électronique 23 autonome constate que le temps imparti depuis la sortie de la poche de sang de l'enceinte à atmosphère contrôlée cellulaire 10 est dépassé, la poche de sang est déclarée douteuse et doit être requalifiée en la remettant en communication avec un dispositif de communication électronique spécialisé 12 ou le quatrième dispositif de communication électronique 20 relié au deuxième ordinateur 13; si la poche de sang n'a pas été utilisée et qu'elle est retournée dans l'enceinte à atmosphère contrôlée cellulaire 10; la procédure de mesure en continu du vieillissement se remet en place après avoir calculé le vieillissement subi par la poche de sang 11 pendant son séjour en atmosphère non contrôlée d'après un modèle de vieillissement préétabli comme celui précédemment décrit et la poche de sang 11 est soit requalifiée soit déqualifiée et envoyée à la destruction 21. Il peut être déterminé plusieurs modèles de vieillissement préétablis permettant de coller au mieux à la réalité du vieillissement suivant les manipulations subies par les poches de sang ; les manipulations concernent, la phase de prélèvement du sang, les phases de centrifugation 14 et séparation 15 des composants, la phase de transport 25 en atmosphère contrôlée entre l'établissement de transfusion sanguine 16 et l'établissement de soins 18.

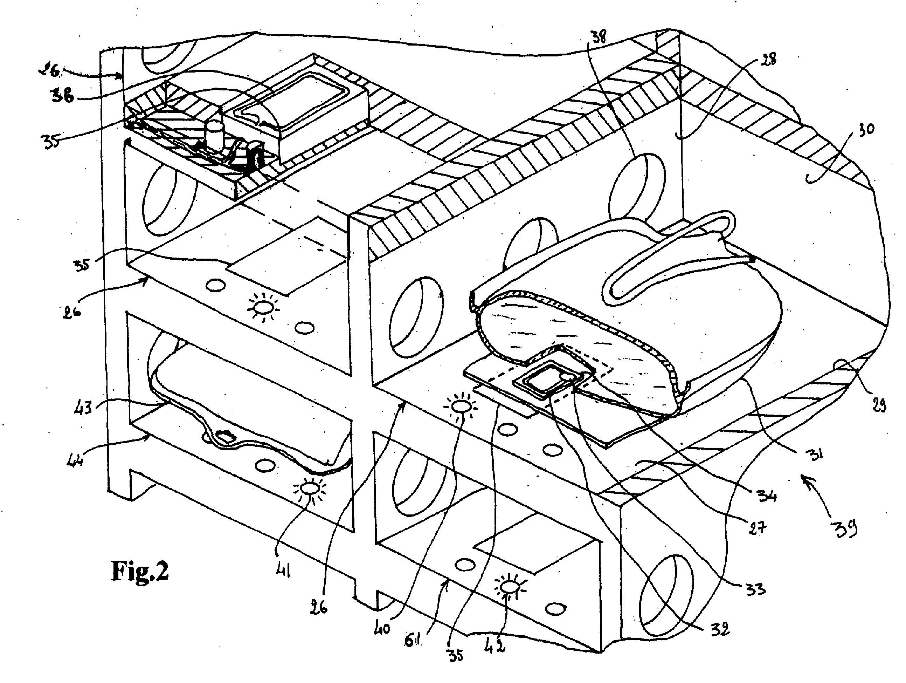

Dans une version préférée d'un dispositif de mise en oeuvre du procédé, le stockage des poches de sang 11 est assuré au niveau des établissements de soins 18 dans une enceinte à atmosphère contrôlée cellulaire 39 formée d'alvéoles 26 (fig.2) sensiblement parallélépipédiques ; les alvéoles 26 comportent notamment une grande face inférieure horizontale 27 bordée de trois petites faces latérales 28,29,30, susceptibles de recevoir et supporter une poche de sang 31 et de la positionner de manière à placer l'antenne en boucle 32 de la puce électronique 33, solidaire d'un support de puce électronique souple 34, en face de l'antenne en boucle 36 d'un dispositif de communication électronique spécialisé 35 intégré dans la grande face horizontale 27 ; cela suppose que le support de puce électronique souple 34 soit situé toujours à la même place sous une étiquette 37, comme précédemment décrit et que cette dernière soit tournée vers la grande face inférieure horizontale 27 ; ces alvéoles 26 sont ajourées par des ouvertures 38 pour permettre une circulation de l'air de réfrigération d'une alvéole 26 à l'autre ; chaque dispositif de communication électronique spécialisé 35 est relié à un même ordinateur qui mémorise tout ou partie des caractéristiques des poches de sang 31 contenues dans les alvéoles 26 et interroge chaque alvéole 26 à intervalle de temps régulier et rapproché pour contrôler la présence effective d'une poche de sang 31 dans une alvéole 26 et calculer son indice de vieillissement afin de l'intégrer dans la puce électronique 33 avec la date et l'heure du calcul.

La gestion du stock de poches de sang dans l'enceinte à atmosphère contrôlée cellulaire 39 est de préférence effectuée par l'ordinateur à partir d'une adresse affectée à chaque alvéole 26 suivant un certain nombre de critères hiérarchisés ; la personne chargée de prélever des poches de sang renseigne les caractéristiques de sa demande sur l'écran de l'ordinateur et notamment décline son identité et l'ordinateur indique en réponse l'adresse des alvéoles 26 contenant les poches de sang 31 correspondant à la demande et aux critères de prélèvement hiérarchisés ; le prélèvement effectif des poches de sang est contrôlé par constatation que l'alvéole 26 est vide ; le prélèvement d'une poche de sang 31 dans une autre alvéole 26 que celles sélectionnées pour le prélèvement déclenchant immédiatement une alarme ; la fin du prélèvement fait l'objet d'un reçu électronique de la part de la personne chargée de faire ledit prélèvement.

Les poches de sang 43 contenues dans l'enceinte à atmosphère contrôlée cellulaire 39 qui atteignent l'indice de vieillissement maximum sont déclassées et signalées sur l'écran de l'ordinateur afin qu'elles soient extraites de leur alvéole pour être détruites.

En ce qui concerne les poches de sang entrantes dans une enceinte à atmosphère contrôlée cellulaire 39, elles sont placées dans les alvéoles 26 vides ; l'ordinateur détecte qu'une alvéole 26 précédemment vide est maintenant occupée car elle peut lire la puce électronique 33 placée sous l'étiquette 37 de la poche de sang 31 ; elle peut en gérer le vieillissement à partir des éléments contenus dans la puce électronique 33, par exemple, dans le cas d'un établissement de soins elle détecte à partir de la lecture de la puce électronique 33 quel a été son parcours ; si la poche de sang 31 arrive de l'établissement de transfusion sanguine 16 (fig.1), elle actualise l'indice de vieillissement et la met en attente de prélèvement pour une opération ; si elle arrive d'une salle d'opération 19, elle doit être requalifiée et repositionnée en fonction de critères hiérarchisés pour être, soit retournée au centre de transfusion sanguine 16, soit déqualifiées et envoyée en destruction 21, soit remise en attente pour une nouvelle opération.

Pour faciliter le remplissage des alvéoles 26 (fig.2) et le prélèvement des poches de sang 31, les alvéoles 26 peuvent être équipées de lampes témoin de couleur, par exemple, une lampe témoin verte 40 qui s'allume lorsque l'alvéole 26 contient une poche de sang 31 sélectionnée pour l'enlèvement une lampe témoin rouge 41 qui s'allume lorsque l'ordinateur détecte que l'alvéole 44 est occupée par une poche de sang 43 périmée ; éventuellement une lame témoin blanche 42 pour une alvéole vide 61; cette dernière lampe témoin blanche 42 permettant de détecter, lorsque l'alvéole est néanmoins occupée par une poche de sang, un dysfonctionnement du dispositif de communication électronique spécialisé 35 ou de la puce électronique 33 ; la lampe témoin verte 40 ou rouge 42 en s'allumant au moment de l'introduction d'une poche de sang 31 ou 43, indique que l'ordinateur a bien pris en compte la gestion de la poche de sang.

Sur le plan pratique les alvéoles 26 d'une enceinte à atmosphère contrôlée cellulaire 39, peuvent être les éléments constitutifs d'un bloc qui est placé dans une enceinte à atmosphère contrôlée non aménagée ; les alvéoles 26 sont de préférence en matière plastique éventuellement armé d'un minimum d'éléments métalliques et sont séparées verticalement d'une distance suffisante pour que l'antenne en boucle 36 du dispositif de communication électronique spécialisé 35 qui est logé dans la grande face horizontale inférieure 27 n'agisse que sur la poche de sang de l'alvéole 26 concernée sans affecter les poches de sang contenues dans les autres alvéoles 26 ; les lampes témoins 40, 41, 42 sont logées dans la grande face inférieure horizontale 27 du côté de l'ouverture latérale de l'alvéole 26.

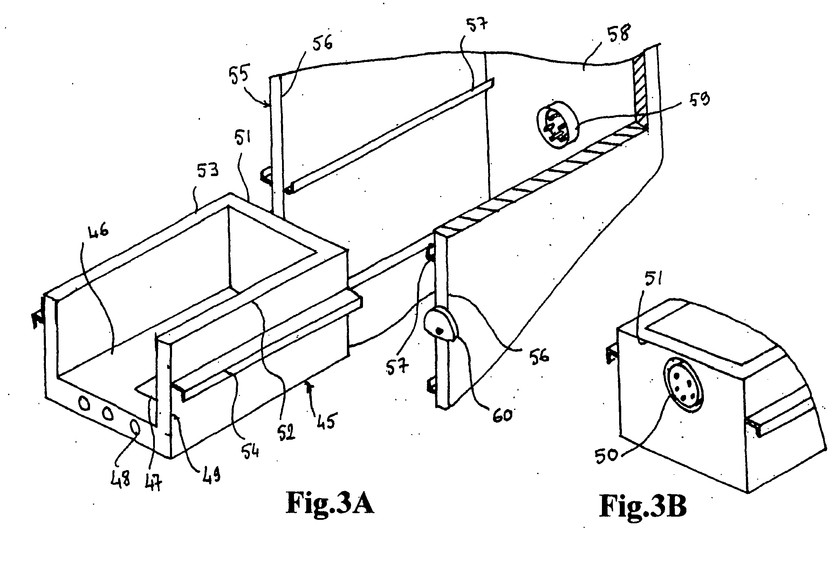

Dans une variante du dispositif de mise en oeuvre du procédé, les alvéoles sont des tiroirs indépendants 45 (fig.3A) formés d'une grande face inférieure 46, intégrant un dispositif de communication électronique spécialisé 47 et les lampes témoins 48, avec un côté 49 constituant l'ouverture pour introduire la poche de sang ; le dispositif de communication électronique spécialisé 47 est relié à un dispositif de connexion primaire 50 (fig.3B) solidaire de la petite face latérale constituant le fond du tiroir 51 (fig.3A) du tiroir indépendant 45 ; les petites faces latérales 52 et 53, qui sont parallèles entre elles comportent extérieurement des rails de guidage 54. Un bloc de stockage 55 destiné à recevoir les tiroirs indépendants 45 est constitué d'échelles 56 placées verticalement parallèlement entre elles à espacement convenable pour recevoir les tiroirs indépendants 45 et comportant à intervalles réguliers des glissières horizontales 57 sur lesquelles les rails de guidage 54 des tiroirs indépendants 45 peuvent coulisser ; le fond de bloc 58 du bloc de stockage 55 comporte des dispositifs de connexion secondaires 59 sur lesquels viennent se fixer les dispositifs de connexion primaires 50 des tiroirs indépendants 45 ; les prises mâles 59 sont reliées à un ordinateur ; la partie avant des échelles 56 peut comporter un dispositif de verrouillage 60 des tiroirs indépendants 45 en place.

## Revendications

1. Procédé de qualification, requalification et déqualification d'une poche de sang (11,31,43,62) par la détermination et le suivi de l'évolution d'un indice de vieillissement de ladite poche de sang, indiquant que cette dernière peut être transfusée ou non sur un patient et sur laquelle est fixée à demeure une puce électronique (33) équipée d'une antenne en boucle (32) susceptible de communiquer avec un dispositif de communication électronique (2,8,12,17,20,23,35) équipé lui-même d'une antenne en boucle (36) relié à un ordinateur (9,13) ou à un dispositif de calcul simplifié (24), selon lequel l'indice de vieillissement est établi à partir d'un instant initial déterminé par un prélèvement de sang sur un donneur de sang dans un établissement de transfusion sanguine (16) et inscrit dans la puce électronique (33) à l'aide d'un dispositif de communication électronique (2) équipant un peson-agitateur (1), la valeur de l'indice de vieillissement évoluant progressivement en fonction notamment du temps et des conditions environnementales vers une valeur maximale qui, lorsqu'elle est atteinte, entraîne la déqualification et la destruction de la poche de sang, la valeur de l'indice de vieillissement étant calculée entre deux instants successifs constituant une période en mettant la puce électronique (33) en relation avec un des dispositifs de communication électronique (8,12,17,20,23,35) lui-même relié à un ordinateur (9,13), en utilisant des modèles de vieillissement préétablis caractérisant la période considérée et qui sont établis expérimentalement à partir de formules empiriques prenant en compte le résultat d'analyses bactériologiques, la valeur de l'indice de vieillissement au début de la période, la durée de la période et les caractéristiques de l'environnement de la poche de sang (11,31,43,62) pendant la période considérée, l'indice de vieillissement étant calculé notamment lors de l'entrée et de la sortie d'une enceinte à atmosphère contrôlée (7,10) et au moment de la transfusion à un patient dans un établissement de soins (16) et la nouvelle valeur de l'indice de vieillissement étant inscrite dans la puce électronique (33) à chaque calcul.

2. Procédé de détermination d'un indice de vieillissement selon la revendication 1, **caractérisé en ce que** ce dernier est calculé au moment où l'on introduit la poche de sang (18) dans l'enceinte à atmosphère contrôlée (7), de l'établissement de transfusion sanguine (16), en utilisant un troisième dispositif de communication électronique (17) relié au premier ordinateur (9), l'indice de vieillissement étant recalculé éventuellement lors du contrôle statistique du stock de poches de sang et au moment où l'on extrait la poche de sang (11) pour l'envoyer vers l'établissement de soins (18), en utilisant le deuxième dispositif de communication électronique (8) relié au premier ordinateur (9).

3. Procédé de détermination d'un indice de vieillissement selon la revendication 1. **caractérisé en ce que** ce dernier est calculé automatiquement, à intervalles de temps réguliers et rapprochés, dans une enceinte à atmosphère contrôlée cellulaire (10,39), où la puce électronique (33) de chaque poche de sang (11,31,43) est mise en relation permanente avec un dispositif de communication électronique spécialisé (12,35) relié à un deuxième ordinateur (13), de manière que la poche de sang (11) puisse être prélevée à tout moment en ayant inscrit dans sa puce électronique (33) son indice de vieillissement à jour, pour être dirigée vers une salle d'opération (19) où l'indice de vieillissement est établi avant transfusion vers le patient grâce à un quatrième dispositif de communication électronique (20) relié au deuxième ordinateur (13), toute poche de sang qui n'est pas utilisée étant retournée dans l'enceinte à atmosphère contrôlée cellulaire (10) et même vers l'établissement de transfusion sanguine (16).

4. Procédé suivant les revendications 1 à 3, **caractérisé en ce qu'**au moment de la transfusion, la qualification de la poche de sang peut être contrôlée grâce à un cinquième dispositif de communication électronique (23) autonome relié à un dispositif de calcul simplifié (24) susceptible de comparer le temps de séjour de la poche de sang en dehors de l'enceinte à atmosphère contrôlée cellulaire (10,39) avec un temps imparti auquel correspond une durée de vie résiduelle en atmosphère non contrôlée hors d'une enceinte à atmosphère contrôlée, inscrit dans la puce électronique (33) lors de son séjour dans l'enceinte à atmosphère contrôlée cellulaire (10,39), durée de vie résiduelle au-delà de laquelle la poche de sang (11) n'est plus transfusable sans requalification par un dispositif de communication électronique (17,12,20) relié à un ordinateur (9,13).

5. Procédé selon les revendications 1 à 4, **caractérisé en ce que** la gestion du stock de poches de sang (31,43) dans l'enceinte à atmosphère contrôlée cellulaire (39) est effectuée par le deuxième ordinateur (13) à partir d'une adresse affectée à des alvéoles(26) contenant les poches de sang et suivant un certain nombre de critères hiérarchisés, la personne chargée de prélever des poches de sang renseignant les caractéristiques de sa demande sur l'écran de l'ordinateur et déclinant son identité, l'ordinateur (13) indiquant en réponse l'adresse des alvéoles (26) correspondant aux caractéristiques demandées et aux critères de prélèvement hiérarchisés.

6. Procédé selon les revendications 1 à 4, **caractérisé en ce que** la valeur de l'indice de vieillissement de la poche de sang (11,43) située dans une enceinte à atmosphère contrôlée cellulaire (10,39) approche de sa valeur maximum et que sa durée de vie conduirait à dépasser ladite valeur maximum, la poche de sang (11,43) est disqualifiée et son adresse signalée par l'ordinateur (13) pour être détruite.

7. Dispositif pour la mise en oeuvre du procédé selon les revendication 1 à 6, **caractérisé en ce que** les alvéoles de l'enceinte à atmosphère contrôlée cellulaire (39) de stockage des poches de sang (11) comportent une grande face inférieure horizontale (27) susceptible de supporter une poche de sang (31) de manière à placer l'antenne en boucle (32) de la puce électronique (33) en face de l'antenne en boucle (36) d'un dispositif de communication électronique spécialisé (35) intégré dans la grande face horizontale (27), chaque dispositif de communication électronique spécialisé (35) étant relié à un même deuxième ordinateur (13) qui mémorise tout ou partie des caractéristiques des poches de sang (31) contenues dans les alvéoles (26) et interroge chaque alvéole (26) à intervalle de temps régulier et rapproché pour contrôler la présence effective d'une poche de sang (31,43) dans une alvéole (26,44) et calculer son indice de vieillissement afin de l'intégrer dans la puce électronique (33) avec la date et l'heure du calcul.

8. Dispositif selon la revendication 7, **caractérisé en ce que** les alvéoles sont des tiroirs indépendants (45) formés d'une grande face inférieure (46), intégrant un dispositif de communication électronique spécialisé (47) relié à un dispositif de connexion primaire (50) solidaire d'une petite face latérale constituant le fond de tiroir (51), des petites faces latérales (52,53) comportant extérieurement des rails de guidage (54) coopérant avec des glissières horizontales (57) solidaires d'échelles (56) formant un bloc de stockage (55) dont le fond de bloc (58) comporte des dispositifs de connexion secondaires (59), sur lesquels viennent se fixer les dispositifs de connexion primaires (50), reliés au deuxième ordinateur 13.

9. Dispositf selon la revendication 7, **caractérisé en ce que** les alvéoles (26,44,61) comportent des lampes témoins (40,41,42), indiquant respectivement les alvéoles (26) contenant une poche de sang (31) sélectionnée par le deuxième ordinateur (13) pour être utilisée, les alvéoles (44) contenant une poche de sang (43) déqualifiée destinée à la destruction, les alvéoles (61) ne contenant pas de poche de sang ou dont le dispositif de communication électronique spécialisé (35) n'est pas opérationnel.

10. Dispostif selon la revendication 7,**caractérisé en ce que** l'ensemble des alvéoles (26) ou le bloc de stockage (55) équipé des tiroirs indépendants (45) forment un ensemble susceptible d'être placé à l'intérieur d'une enceinte à atmosphère contrôlée non aménagée.

## Claims

1. Method for qualifying, re-qualifying and disqualifying a blood bag (11, 31, 43, 62) by determining and monitoring the evolution of an ageing index on the said blood bag, indicating that the latter may or may not be transfused to a patient and on which is permanently attached a silicon chip (33) fitted with a loop antenna (32) capable of communicating with an electronic communication device (2, 8, 12, 17, 20, 23, 35) also equipped with a loop antenna (36) connected to a computer (9.13) or to a simplified calculating device (24), according to which the ageing index is established beginning at an initial moment when the blood is collected from a donor in a blood transfusion centre (16) and entered on the silicon chip (33) by means of an electronic communication device (2) fitted to a weighing and stirring device (1), the value of the ageing index evolving progressively, notably depending on the time elapsed and environmental conditions, towards a maximum value, which, when it has been reached, leads to the blood bag being disqualified and destroyed, the value of the ageing index being calculated between two successive moments in time that constitute a period by putting the silicon chip (33) in relation with one of the electronic communication devices (8, 12, 17, 20, 23, 35), itself connected to a computer (9.13), using pre-determined ageing models characteristic of the period under consideration and established by experiment on the basis of empirical formulae taking account of bacteriological analyses, the value of the ageing index at the beginning of the period, the duration of the period and the environmental characteristics of the blood bag (11, 31, 43, 62) during the period under consideration. As the ageing index is calculated more particularly when the blood bag enters or leaves a controlled atmosphere chamber (7.10) and when a patient is transfused with the blood that it contains in a health-care institution (16) and the new ageing index value is entered in the silicon chip (33) after each calculation.

2. Method for determining an ageing index as per claim 1, **characterised by** the fact that the index is calculated when the blood bag (18) is put into the controlled atmosphere chamber (7) at the blood transfusion centre (16), using a third electronic communication device (17) connected to the first computer (9), the ageing index being recalculated if needs be when stocks of blood bags are controlled for statistical reasons and when the blood bag (11) is removed to be dispatched to a health-care institution, using the second electronic communication device (8) connected to the first computer (9).

3. Method for determining an ageing index as per claim 1, **characterised by** the fact that the index is calculated automatically at close and regular intervals of time in a cellular controlled atmosphere chamber (10, 39) where the silicon chip (33) on each blood bag (11, 31, 43) is put into permanent relation with a dedicated electronic communication device (12, 35) connected to a second computer (13) so that the blood bag may be removed at any moment after an up-to-date ageing index has been entered on its silicon chip, to be dispatched to an operating theatre (19) where the ageing index is established before a patient is transfused with the blood contained in the blood bag by means of a fourth electronic communication device (20) connected to the second computer (13), any unused blood bag being put back into the cellular controlled atmosphere chamber (10) or even being returned to the blood transfusion centre (16).

4. Method as per claims 1 to 3, **characterised by** the fact that, at the moment of the transfusion, the qualification of the blood bag may be checked by means of a fifth independent electronic communication device (23) connected to a simplified calculating system (24) capable of comparing the length of time spent by the blood bag outside the cellular controlled atmosphere chamber (10, 39) with an allocated length of time, corresponding to the life-span remaining in non-controlled atmosphere outside a controlled atmosphere chamber, entered in the silicon chip (33) while it was stocked in the cellular controlled atmosphere chamber (10, 39), a life-span longer than which the blood bag (11) can no longer be used for transfusions without being re-qualified by an electronic communication device (17,12, 20) connected to a computer (9, 13).

5. Method as per claims 1 to 4, **characterised by** the fact that stock management of the blood bags (31, 43) is carried out inside the cellular controlled atmosphere chamber (39) by a second computer (13) using an address allocated to each compartment (26) containing the blood bags and corresponding to a certain number of hierarchical criteria, the person responsible for removing the blood bags entering the characteristics of his or her requirement on the computer screen and giving his or her identity, the computer (13) indicating in reply the address of the compartments corresponding to the requirements requested and the hierarchical criteria for removal.

6. Method as per claims 1 to 4, **characterised by** the fact that the ageing index value of the blood bag (11, 43) placed in the cellular controlled atmosphere chamber (10, 39) is approaching its maximum value and that its life-span will lead to this maximum value being exceeded, the blood bag (11, 43) is disqualified and its address indicated by the computer (13) for destruction.

7. Device for implementing the method as per claims 1 to 6, **characterised by** the fact that the compartments in the cellular controlled atmosphere chamber (39) for storing the blood bags (11) include a wide horizontal lower surface (27) capable of holding a blood bag (31) in such a way that the loop antenna (32) on the silicon chip (33) is placed opposite the loop antenna (36) of a dedicated electronic communication device (35) integrated into the wide horizontal surface (27), each dedicated electronic communication device (35) being connected to the same second computer (13) that memorises all or part of the characteristics of the blood bags (31) contained in the compartments (26) and consults every compartment at close and regular intervals in time for checking that a blood bag (31, 43) is in fact present in a compartment (26, 44) and calculating its ageing index for incorporating the index in the silicon chip (33) with the time and date of the calculation.

8. Device as per claim 7, **characterised by** the fact that the compartments are independent drawers (45) composed of a wide lower face (46), incorporating a dedicated electronic communication device (47) connected to a primary connecting device (50) attached to the small side face that constitutes the back of the drawer (51), small side faces (52, 53) with guide rails on their outside surface (54), running on horizontal slide-ways (57) attached to rack arrangements (56), which form a storage unit (55) the back of which (58) contains secondary connecting devices (59) to which the primary connecting devices (50) are attached, connected to the second computer (13).

9. Device as per claim 7, **characterised by** the fact that the compartments (26, 44, 61) include indicator lamps (40, 41, 42) respectively indicating the compartments (26) that contain a blood bag (31) selected for use by the second computer, compartments (44) containing a disqualified blood bag (43) to be sent away for destruction, compartments (61) that do not contain a blood bag or whose dedicated electronic communication device (35) is not operational.

10. Device as per claim 7, **characterised by** the fact that all the compartments (26) or the storage unit (55) fitted with independent drawers (45) form a complete unit capable of being put inside a non-equipped controlled atmosphere chamber.

## Patentansprüche

1. Verfahren zum Bewerten, Umbewerten und Ausscheiden eines Blutbeutels (11, 31, 43, 62) durch die Bestimmung und die Verfolgung eines Alterungsindexes des besagten Blutbeutels mit Angabe, daß letzterer zur Transfusion bei einem Patienten verwendet werden kann oder nicht, und auf dem ein mit einer Spulenantenne (32) ausgerüsteter elektronischer Chip (33) auf Dauer befestigt ist, der mit einer elektronischen Kommunikationsvorrichtung (2, 8, 12, 17, 20, 23, 35) kommuniziert, die mit einer Spulenantenne (36) ausgerüstet ist, die mit einem Computer (9, 13) oder einer vereinfachten Rechenvorrichtung (24) verbunden ist nach der der Alterungsindex ab einem ursprünglichen Moment festgelegt wird, der durch eine Blutentnahme von einem Blutspender in einer Einrichtung für Bluttransfusionen (16) bestimmt und in den elektronischen Chip (33) mit einer elektronischen Kommunikationsvorrichtung (2) geschrieben wird, die eine Schnellwaage mit Rührwerk (1) ausrüstet, wobei sich der Wert des Alterungsindexes nach und nach in Abhängigkeit insbesondere von der Zeit und den Umgebungsbedingungen zu einem Höchstwert entwickelt, der, wenn er erreicht ist, die Ausscheidung und die Vernichtung des Blutbeutels mit sich führt, wobei der Wert des Alterungsindexes zwischen zwei aufeinanderfolgenden Momenten berechnet wird, die eine Periode bilden, indem der elektronische Chip (33) in Beziehung zu einer der elektronischen Kommunikationsvorrichtungen (8, 12, 17, 20, 23, 35) gesetzt wird, die selbst mit einem Computer (9, 13) verbunden ist, unter Benutzung von vorerstellten Alterungsmodellen, die die betrachtete Periode kennzeichnen, und die experimentell ausgehend von empirischen Formeln erstellt werden, die das Ergebnis von bakteriologischen Auswertungen, den Wert des Alterungsindexes zu Beginn der Periode, die Dauer der Periode und die Merkmale der Umgebung des Blutbeutels (11, 31, 43, 62) während der betrachteten Periode berücksichtigen, wobei der Alterungsindex insbesondere beim Eintreten und Verlassen eines Raums mit Schutzatmosphäre (7, 10) und im Moment der Transfusion eines Patienten in einer Pflegeeinrichtung (16) berechnet wird, und wobei der neue Wert des Alterungsindexes bei jeder Berechnung in den elektronischen Chip (33) eingeschrieben wird.

2. Verfahren zur Bestimmung eines Alterungsindexes nach Anspruch 1, **dadurch gekennzeichnet, daß** letzterer in dem Moment berechnet wird, in dem man den Blutbeutel (18) in den Raum mit Schutzatmosphäre (7) der Einrichtung für Bluttransfusionen (16) einbringt, indem eine dritte elektronische Kommunikationsvorrichtung (17) benutzt wird, die mit dem ersten Computer (9) verbunden ist, wobei der Alterungsindex eventuell bei der statistischen Kontrolle des Bestands an Blutbeuteln und in dem Moment neu berechnet wird, in dem der Blutbeutel (11) herausgenommen wird, um ihn zur Pflegeeinrichtung (18) zu schicken, indem die zweite elektronische Kommunikationsvorrichtung (8) benutzt wird, die mit dem ersten Computer (9) verbunden ist.

3. Verfahren zur Bestimmung eines Alterungsindexes nach Anspruch 1, **dadurch gekennzeichnet, daß** letzterer automatisch in regelmäßigen und kurzen Zeitabständen in einem Zellraum mit Schutzatmosphäre (10, 39) berechnet wird, wo der elektronische Chip (33) jedes Blutbeutels (11, 31, 43) in ständige Verbindung mit einer spezialisierten elektronischen Kommunikationsvorrichtung (12, 35) gebracht wird, die mit einem zweiten Computer (13) verbunden ist, so daß der Blutbeutel (11) jederzeit entnommen werden kann, wobei in seinen elektronischen Chip (33) sein aktueller Alterungsindex eingeschrieben wurde, damit er in einen Operationssaal (19) geleitet wird, wo der Alterungsindex vor der Transfusion des Patienten durch eine vierte elektronische Kommunikationsvorrichtung (20) festgestellt wird, die mit dem zweiten Computer (13) verbunden ist, wobei jeder Blutbeutel, der nicht verwendet wird, in den Zellraum mit Schutzatmosphäre (10) und sogar an die Einrichtung für Bluttransfusionen (16) zurückgeschickt wird.

4. Verfahren nach einem beliebigen der vorstehenden Ansprüche 1 bis 3, **dadurch gekennzeichnet, daß** im Moment der Transfusion die Bewertung des Blutbeutels durch eine fünfte autonome elektronische Kommunikationsvorrichtung (23) kontrolliert werden kann, die mit einer vereinfachten Rechenvorrichtung (24) verbunden ist, die die Dauer des Aufenthalts des Blutbeutels außerhalb des Zellraums mit Schutzatmosphäre (10, 39) mit einer vorgegebenen Zeit vergleicht, der eine Restlebensdauer ohne Schutzatmosphäre außerhalb eines Raums mit Schutzatmosphäre entspricht, die bei seinem Aufenthalt im Zellraum mit Schutzatmosphäre (10, 39) in den elektronischen Chip (33) eingeschrieben wird, und zwar die Restlebensdauer, über die hinaus der Blutbeutel (11) ohne Neubewertung durch eine elektronische Kommunikationsvorrichtung (17, 12, 20), die mit einem Computer (9, 13) verbunden ist, nicht mehr zur Transfusion verwendet werden kann.

5. Verfahren nach einem beliebigen der vorstehenden Ansprüche 1 bis 4, **dadurch gekennzeichnet, daß** das Bestandsmanagement der Blutbeutel (31, 43) im Zellraum mit Schutzatmosphäre (39) vom zweiten Computer (13) ab einer Adresse gesteuert wird, die Wabenfächern (26) zugeordnet ist, sowie nach einer gewissen Reihe hierarchisch geordneter Kriterien, wobei die mit der Entnahme der Blutbeutel beauftragte Person die Merkmale ihrer Anfrage sowie Angaben zu ihrer Person im Bildschirm des Computers einträgt, wonach der Computer (13) als Antwort die Adresse der Wabenfächer (26) angibt, die den angeforderten Merkmalen und den hierarchisch geordneten Kriterien der Entnahme entsprechen.

6. Verfahren nach einem beliebigen der vorstehenden Ansprüche 1 bis 4, **dadurch gekennzeichnet, daß,** wenn sich der Wert des Alterungsindexes des Blutbeutels (11, 43), der sich in einem Zellraum mit Schutzatmosphäre (10, 39) befindet, seinem Höchstwert annähert und seine Lebensdauer zum Überschreiten des besagten Höchstwerts führen würde, der Blutbeutel (11, 43) ausgeschieden wird und der Computer (13) seine Adresse zur Vernichtung angibt.

7. Vorrichtung für den Einsatz des Verfahrens nach einem beliebigen der vorstehenden Ansprüche 1 bis 6, **dadurch gekennzeichnet, daß** die Wabenfächer des Zellraums mit Schutzatmosphäre (39) für die Lagerung der Blutbeutel (11) eine große untere horizontale Fläche (27) umfassen, die einen Blutbeutel (31) so tragen kann, daß die Spulenantenne (32) des elektronischen Chips (33) gegenüber der Spulenantenne (36) einer speziellen elektronischen Kommunikationsvorrichtung (35) plaziert wird, die in die große horizontale Fläche (27) integriert ist, wobei jede spezielle elektronische Kommunikationsvorrichtung (35) mit einem gleichen zweiten Computer (13) verbunden ist, der alle oder einen Teil der Merkmale der Blutbeutel (31) speichert, die in den Wabenfächern (26) enthalten sind und jedes Wabenfach (26) in regelmäßigen und kurzen Zeitabständen abfragt, um das effektive Vorhandensein eines Blutbeutels (31, 43) in einem Wabenfach (26, 44) zu kontrollieren und seinen Alterungsindex zu berechnen, um ihn zusammen mit dem Datum und der Uhrzeit der Berechnung in den elektronischen Chip (33) einzugeben.

8. Vorrichtung nach Anspruch 7, **dadurch gekennzeichnet, daß** die Wabenfächer unabhängige Schubladen (45) sind, die aus einer großen unteren Fläche (46) gebildet werden, eine spezielle elektronische Kommunikationsvorrichtung (47) integrieren, die mit einer primären Anschlußvorrichtung (50) verbunden ist und fest mit einer kleinen Seitenfläche verbunden ist, die die Rückwand der Schublade (51) bildet, wobei kleine Seitenflächen (52, 53), die außen Führungsschienen (54) umfassen, die mit horizontalen Schiebern (57) zusammenwirken, die mit Kantenflächen (56) fest verbunden sind und einen Aufbewahrungsblock (55) bilden, dessen Blockrückwand (58) sekundäre Anschlußvorrichtungen (59) umfaßt, auf denen sich die primären Anschlußvorrichtungen (50) befestigen, die mit dem zweiten Computer (13) verbunden sind.

9. Vorrichtung nach Anspruch 7, **dadurch gekennzeichnet, daß** die Wabenfächer (26, 44, 61) Anzeigelampen (40, 41, 42) umfassen, die jeweils das Wabenfach (26) angeben, das einen Blutbeutel (31) enthält, der vom zweiten Computer (13) zur Verwendung gewählt wurde, die Wabenfächer (44), die einen ausgeschiedenen Blutbeutel (43) enthalten, der zur Vernichtung bestimmt ist, die Wabenfächer (61), die keinen Blutbeutel enthalten oder deren spezielle elektronische Kommunikationsvorrichtung (35) nicht betriebsbereit ist.

10. Vorrichtung nach Anspruch 7, **dadurch gekennzeichnet, daß** alle Wabenfächer (26) oder der mit unabhängigen Schubladen (45) ausgerüstete Aufbewahrungsblock (55) ein Ensemble bilden, das in einem einrichtungslosen Raum mit Schutzatmosphäre aufgestellt werden kann.
